(19) Europäisches Patentamt
European Patent Office
Office européen des brevets

(11) EP 4 159 185 A1

(12) # EUROPEAN PATENT APPLICATION

(43) Date of publication:
**05.04.2023 Bulletin 2023/14**

(21) Application number: **21200135.8**

(22) Date of filing: **30.09.2021**

(51) International Patent Classification (IPC):
***A61K 8/02*** *(2006.01)* ***A61K 8/24*** *(2006.01)*
***A61K 8/41*** *(2006.01)* ***A61Q 5/10*** *(2006.01)*

(52) Cooperative Patent Classification (CPC):
**A61Q 5/10; A61K 8/022; A61K 8/24; A61K 8/411;**
A61K 2800/31; A61K 2800/882

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kao Corporation**
**Chuo-ku**
**Tokyo 103-8210 (JP)**

(72) Inventors:
- **Nakamura, Takahito**
  **Tokyo 131-8501 (JP)**
- **Hiruma, Daisuke**
  **Tokyo 131-8501 (JP)**

(74) Representative: **Hoffmann Eitle**
**Patent- und Rechtsanwälte PartmbB**
**Arabellastraße 30**
**81925 München (DE)**

(54) **COMPOSITION COMPRISING AN OXIDATIVE DYE AND ALKALIZING AGENT**

(57) The present invention is directed to dyeing composition A for keratin fibers, preferably for human keratin fibers, more preferably for human hair, comprising a) 1,4-diamino-2-methoxymethyl-benzene, and/or its salt(s), and b) one or more alkalizing agent(s), wherein the total concentration of water is 10% by weight or less, calculated to the total weight of composition A.

EP 4 159 185 A1

## Description

### Field of the invention

The present invention relates to a composition for dyeing of keratin fibers with an oxidative dye and alkalizing agent. Furthermore, a dyeing product and dyeing method is disclosed.

### Background of the invention

Oxidative dyes possess superior dyeing abilities and grey coverage, but in case of suboptimal conditions, their dyeing performance decreases. Once ready-to-use mixtures are prepared, the color mixtures become dark and may quickly lose their ability to dye keratin fibers.

Cosmetic industry has tackled these problems by excluding air oxygen from the compositions during storage. For example, many dyeing compositions are individually packaged or, if offered as multi-use device, supplied with a protective gas such as nitrogen or carbon dioxide. In addition, formulation parameters of oxidative hair dyes are carefully assessed.

A particular challenge is the stable formulation of dyeing products with low amount of water.

As the prior art has not satisfactorily solved the above challenges, there is a real need to develop dyeing compositions, which are stable over time.

### Summary of the invention

The first object of the present invention is a dyeing composition A for keratin fibers, preferably for human keratin fibers, more preferably for human hair, comprising

a) 1,4-diamino-2-methoxymethyl-benzene, and/or its salt(s), and

b) one or more alkalizing agent(s),

wherein the total concentration of water is 10% by weight or less, calculated to the total weight of composition A.

The second object of the present invention is a dyeing product for keratin fibers, preferably human keratin fibers, more preferably human hair, comprising:

- composition A as defined in any above,
- a composition B, preferably comprising one or more oxidizing agent(s).

The third object of the present invention is a method for dyeing keratin fibers, preferably human keratin fibers, more preferably human hair, comprising the steps of:

i) mixing the composition A as defined above with an aqueous composition, preferably with composition B as defined above, to yield a ready-to-use composition having a pH in the range of 7 to 12,

ii) applying the ready-to-use composition onto keratin fibers and leaving it for a time period in the range of 1 min to 60 min,

iii) optionally rinsing-off the keratin fibers and optionally drying the keratin fibers.

The fourth object of the present invention is use of the composition as defined above for dyeing of keratin fibers, preferably for dyeing of human keratin fibers, more preferably for dyeing of human hair.

### Detailed description of the invention

Inventors of the present invention have unexpectedly found out that composition A according to claim 1 had superior dyeing intensity independently of its storage conditions. Furthermore, even after preparation of the ready-to-use mixture, the dyeing ability could be maintained over a certain storage time. In addition, it delivered a superior dyeing intensity and improved wash fastness, in particular on damaged keratin fibers. A further unexpected and surprising effect of the present invention is the reduction of heat generation of the hair dyeing mixture, thereby improving safety and

comfort for the client.

**Composition for dyeing**

The present invention is directed to a dyeing composition A for keratin fibers, preferably for human keratin fibers, more preferably for human hair, comprising

a) 1,4-diamino-2-methoxymethyl-benzene, and/or its salt(s), and

b) one or more alkalizing agent(s),

wherein the total concentration of water is 10% by weight or less, calculated to the total weight of composition A.

Suitable salts of compound(s) according to group a) is/are sulfate salt, hydrochloride salt, hydrobromide salt, nitrate salt, phosphate salt, hydrogen phosphate salt, dihydrogen phosphate salt, methosulfate salt, citrate salt, succinate salt, tartrate salt, lactate salt, tosylate salt, benzenesulfonate salt, acetate salt, and/or their mixtures, preferably it/they is/are a sulfate salt and/or a hydrochloride salt, and/or their mixtures, more preferably it is a sulfate salt, form the viewpoint of speed of dissolution.

It is preferred from the viewpoint of dyeing intensity that the total concentration of compound(s) according to group a) is 0.01% by weight or more, more preferably 0.03% by weight or more, still more preferably 0.05% by weight or more, still further more preferably 0.1% by weight or more, calculated to the total weight of composition A.

It is preferred from the viewpoint of dissolution rate that the total concentration of compound(s) according to group a) is 65% by weight or less, preferably 50% by weight or less, further more preferably 30% by weight or less, still further more preferably 20% by weight or less, calculated to the total weight of composition A.

For attaining the above-mentioned effects, it is preferred that the total concentration of one or more compound(s) according to group a) is/are in the range of 0.01% to 65% by weight, preferably in the range of 0.03% to 50% by weight, more preferably in the range of 0.05% to 30% by weight, still more preferably in the range of 0.1% to 20% by weight, calculated to the total weight of composition A.

It is further preferred from the viewpoint of stability that the total concentration of water is 5% by weight or less, preferably 1% by weight or less, more preferably 0.1% by weight or less, calculated to the total weight of composition A, still more preferably composition A is anhydrous.

The term 'anhydrous' denotes a composition that is free of added water. However, this does not exclude bound water such as residual moisture or crystal water.

It is preferred from the viewpoint of dyeing performance that one or more compound(s) according to group b) is/are one or more inorganic alkalizing agent(s), preferably one or more metal salt of silicate, metasilicate, disilicate, hydroxide, or phosphate, more preferably one or more sodium salt(s) or potassium salt(s) of silicate, metasilicate, disilicate, hydroxide or phosphate, still more preferably trisodium phosphate.

It is preferred from the viewpoint of dye stability that one or more compound(s) according to group b) is/are selected from one or more metal salt of silicate, metasilicate, disilicate, hydroxide, or phosphate, more preferably one or more sodium salt(s) or potassium salt(s) of silicate, metasilicate, disilicate, hydroxide, or phosphate, still more preferably trisodium phosphate or tripotassium phosphate, and/or their mixtures.

Further preferably, one or more compound(s) according to group b) is/are selected from sodium silicate, potassium silicate, sodium disilicate, potassium disilicate, sodium dihydrogen phosphate, disodium hydrogen phosphate, potassium dihydrogen phosphate, potassium hydrogen phosphate, trisodium phosphate, tripotassium phosphate, and/or their mixtures.

The most preferred compound(s) according to group b) is/are trisodium phosphate, tripotassium phosphate, and/or their mixtures.

In addition, composition A may comprise one or more organic alkalizing agent(s) and/or its/their salt(s) is one or more organic alkyl and/or alkanol amines and/or their salt(s) according to the following general structure:

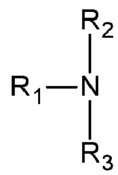

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or their salts,

and/or their mixtures.

Preferably, one or more organic alkalizing agent(s) are selected from alkyl and/or alkanolamine(s) and/or its/their salt(s), more preferably they/it is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane and/or its/their salt(s), and/or their mixtures, from the viewpoint of providing alkalinity and cosmetic safety as well as their low odor.

The most preferred additional alkalizing agent(s) is/are selected from monoethanolamine, 2-amino-2-methylpropanol, tris-(hydroxymethyl)-aminomethane, and/or its/their salt(s), and/or their mixtures, from the viewpoint of providing alkalinity and cosmetic safety.

It is preferred from the viewpoint of dyeing intensity that the total concentration of compound(s) according to group b), preferably the total concentration of inorganic alkalizing agents, more preferably the total concentration of one or more metal salt(s) of silicate, metasilicate, disilicate, or phosphate, still more preferably the total concentration of trisodium phosphate or tripotassium phosphate, is 1% by weight or more, more preferably 2% by weight or more, further more preferably 5% by weight or more, still further more preferably 10% by weight or more, calculated to the total weight of composition A.

It is preferred from the viewpoint of cosmetic safety that the total concentration of compound(s) according to group b), preferably the total concentration of inorganic alkalizing agents, more preferably the total concentration of one or more metal salt(s) of silicate, metasilicate, disilicate, or phosphate, still more preferably the total concentration of trisodium phosphate or tripotassium phosphate, is 80% by weight or less, more preferably 70% by weight or less, further more preferably 65% by weight or less, calculated to the total weight of composition A.

For attaining the above-mentioned effects, it is preferred that the total concentration of compound(s) according to group b), preferably the total concentration of inorganic alkalizing agents, more preferably the total concentration of one or more metal salt(s) of silicate, metasilicate, disilicate, or phosphate, still more preferably the total concentration of trisodium phosphate or tripotassium phosphate, is in the range 1% to 80% by weight, preferably in the range of 2% to 70% by weight, more preferably in the range of 5% to 65% by weight, more preferably in the range of 10% to 65% by weight, calculated to the total weight of composition A.

It is further preferred from the viewpoint of color shading that composition A comprises one or more oxidative dye precursor or oxidative dye coupler different from group a) as compound(s) according to group c), preferably 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorophenol, 1,3-bis-(2,4-diaminophenoxy)-propane, 2-bis(2-hydroxyethyl)-aminotoluene, 2-amino-5-methylphenol, resorcinol, 2-methylresorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 2,4-diaminophenoxyethanol, 1,3-diamino- benzene, 1-amino-3-(2'-hydroxyethylamino)benzene, 1-amino-3-[bis(2'-hydroxy-ethyl) amino]benzene, $\alpha$-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)-benzene, and/or their salt(s), and/or their mixtures, preferably one or more compound(s) according to group b) is/are selected from 4-chlororesorcinol, 2-methylresorcinol, 1,3-bis-(2,4-diaminophenoxy)-propane, 2-methyl-5-amino-6-chlorophenol, 2-amino-3-hydroxypyridine, 5-amino-6-chloro-o-cresol, 3-amino-2,4-dichlorophenol, 2,4,5,6-tetraaminopyrimidine, 2,5,6-triamino-5-pyrimidinol, and/or their salt(s), and/or their mixtures, more preferably 1,3-bis-(2,4-diamino-phenoxy)-propane, 2,4-diaminophenoxyethanol, 2-amino-4-hydroxyethylaminoanisole, 5-amino-6-chloro-o-cresol, 3-amino-2,4-dichlorophenol, and/or their salt(s), and/or their mixtures.

It is preferred from the viewpoint of dyeing intensity that the total concentration of compound(s) according to group c) is 0.01% by weight or more, preferably 0.05% by weight or more, further more preferably 0.08% by weight or more, calculated to the total weight of composition A.

It is preferred from the viewpoint of cosmetic safety that the total concentration of compound(s) according to group c) is 20% by weight or less, preferably 15% by weight or less, further more preferably 10% by weight or less, still further more preferably 5% by weight or less, still further more preferably 3.5% by weight or less, calculated to the total weight of composition A.

For attaining the above-mentioned effects, it is preferred that the total concentration of compound(s) according to group c) is in the range of 0.01% to 20% by weight, preferably in the range of 0.05% to 15% by weight, more preferably in the range of 0.08% to 10% by weight, still more preferably in the range of 0.08% to 5% by weight, still further more preferably in the range of 0.08% to 3.5% by weight, calculated to the total weight of composition A.

It is preferred from the viewpoint of color shades that the weight ratio of compound(s) according to group a) to compound(s) according to group c) is 0.2 or more, more preferably 0.3 or more, still more preferably 0.5 or more.

It is preferred from the viewpoint of color shades that the weight ratio of compound(s) according to group a) to

compound(s) according to group c) is 5 or less, more preferably 3 or less, still more preferably 2 or less.

For attaining the above-mentioned effects, it is preferred that the weight ratio of compound(s) according to group a) to compound(s) according to group c) is in the range of 0.2 to 5, preferably in the range of 0.3 to 3, more preferably in the range of 0.5 to 2.

It is further preferred from the viewpoint of ready-to-use mixture stability and hair damage that the total concentration of carbonates, hydrogen carbonates, bicarbonates, percarbonates, borates, and perborates in composition A is less than 10% by weight, preferably less than 5% by weight, more preferably less than 1% by weight, still more preferably less than 0.1% by weight, calculated to the total weight of composition A, still more preferably it is free of carbonates, hydrogen carbonates, bicarbonates, and percarbonates.

**Direct dyes**

It is further preferred from the viewpoint of dyeing intensity the composition A comprises one or more direct dye(s).

Suitably, one or more direct dye(s) may be HC Blue 18, HC Red 18, HC Yellow 16, Disperse Black 9, Acid Yellow 1, 2-amino-6-chloro-4-nitrophenol, and/or their salt(s), and/or their mixtures.

Suitably, the total concentration of direct dye(s) is 0.01% by weight or more, preferably 0.05% by weight or more, more preferably 0.1% by weight or more, calculated to the total weight of composition A.

Suitably, the total concentration of direct dyes is 20% by weight or less, preferably 15% by weight or less, more preferably 10% by weight or less, calculated to the total weight of composition A.

Suitably, the total concentration of direct dyes in composition A is in the range of 0.01% to 20% by weight is in the range of 0.05% to 15% by weight, more preferably in the range of 0.1% to 10% by weight, calculated to the total weight of composition A.

**Compound(s) according to group d)**

It is preferred from the viewpoint of composition stability and convenience of use that composition A comprises one or more pulverulent excipient as compound(s) according to group d).

The term ’excipient’ denotes a compound, which may act as filling material and dispersant for the other compounds of composition A and does not react with the dyes or the alkalizing agent, and, thus, confers the powder a certain degree of storage stability over an extended period of time.

Composition A of the present invention may comprise an organic and/or an inorganic pulverulent excipient in which the dyes are dispersed.

Suitable organic and/or an inorganic pulverulent excipients are, for example, diatomaceous earth, kaolin, bentonite, starch especially corn, tapioca, rice, wheat and potato, nylon powder, montmorillonit, gypsum, sawdust and perlite, more preferably it is corn starch, from the viewpoint of stability.

Preferably, the total concentration of compound(s) according group d), more preferably the total concentration of organic and/or an inorganic pulverulent excipients, is 30% by weight or more, more preferably 50% by weight or more, further more preferably 60% by weight or more, still further more preferably 65% by weight or more, even further more preferably 70% by weight or more, even more preferably 75% by weight or more, calculated to the total weight of composition A, from the viewpoint of achieving good dispersability of the dyes in the powder and quick dissolution of the powder.

Preferably, the total concentration of compound(s) according group d), more preferably the total concentration of organic and/or an inorganic pulverulent excipients, is 98% by weight or less, more preferably 95% by weight or less, further more preferably 90% by weight or less, calculated to the total weight of composition A, from the viewpoint of achieving good dispersability of the dyes in the powder and formulation freedom.

For attaining the above mentioned effects, it is preferred that the total concentration of compound(s) according group d), more preferably the total concentration of organic and/or an inorganic pulverulent excipients, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

**Powder composition**

In one aspect of the present invention, composition A may be a solid composition at 25°C and atmospheric pressure, preferably a powder composition or a pellet composition or a tablet composition or capsule composition, more preferably a hair dyeing powder composition or a hair dyeing pellet composition or a hair dyeing tablet composition, still more preferably it is a hair dyeing powder composition.

The term 'powder' denotes a solid composition at 25°C and atmospheric pressure. Preferably, form the viewpoint of stability, composition A is an anhydrous powder composition, from the viewpoint of stability.
Suitably, composition A is an anhydrous powder composition, preferably an anhydrous hair dyeing powder composition.

**Lipophilic compounds as compounds according to group e)**

Compositions A and/or B may comprise one or more lipophilic compound(s) as compound(s) according to group e).
Preferably, compounds according to group e) are selected from $C_{12}$ to $C_{22}$ fatty alcohols, esters of $C_3$ to $C_{22}$ alcohols with $C_{12}$ to $C_{22}$ fatty acids, $C_8$ to $C_{22}$ fatty acids, vegetable oils, and/or silicones, and/or hydrocarbon-based products, and/or their mixtures, from the viewpoint of cosmetic compatibility.
Suitable $C_{12}$ to $C_{22}$ fatty alcohols are are myristyl alcohol, cetyl alcohol, stearyl alcohol, behenyl alcohol, and cetearyl alcohol.
Suitable esters of $C_3$ to $C_{22}$ alcohols with $C_{12}$ to $C_{22}$ fatty acids are isopropyl myristate, isopropyl palmitate, and myristyl myristate.
Suitable $C_8$ to $C_{22}$ fatty acids are oleic acid, linoleic acid, and palmitic acid.
Suitable vegetable oils are olive oil, almond oil, sunflower oil, and argan oil.
Suitable silicones are non-aminated and/or aminated silicones. The latter are commonly known as amodimethicones.
It is preferred from the viewpoint of forming a stable composition and user friendliness that the total concentration of compounds according to group e) is 1% by weight or more, more preferably 2% by weight or more, further more preferably 3% by weight or more, calculated to the total weight of each of the compositions A and/or B.
It is preferred from the viewpoint of forming a stable composition that the total concentration of compounds according to group e) is 20% by weight or less, more preferably 15% by weight or less, further more preferably 12% by weight or less, calculated to the total weight of each of the compositions A and/or B.
For attaining the above-mentioned effects, the total concentration of compounds according to group e) is in the range of 1% to 20% by weight, preferably in the range of 2% to 15% by weight, more preferably in the range of 3% to 12% by weight, calculated to the total weight of each of the compositions A and/or B.

**Liquid composition**

In another aspect of the present invention, composition A of the present invention is a liquid composition at 25°C and atmospheric pressure comprising one or more organic solvent(s) as compound(s) according to group f), calculated to the total weight of composition A. Preferably, composition A is anhydrous, from the viewpoint of dye stability.
The term 'liquid' denotes a physical state at 25°C and atmospheric pressure, i.e., composition A is liquid at room temperature.
The term 'anhydrous' denotes a composition A, which is free of added water. This does not exclude the presence of residual moisture from air or crystal water bound to ingredients.
For this aspect of the present invention, composition A may comprise one or more organic solvent(s).
The organic solvent(s) may be selected to dissolve the dyes. Preferred solvents are mono-, di-, and trivalent alcohols and/or their mixtures.
Preferred mono-, di-, and trivalent alcohols from the viewpoint of cosmetic safety and dissolution capacity are ethanol, n-propanol, isopropanol, propylene glycol, ethylene glycol, benzyl alcohol, phenoxyethanol, and glycerol, and/or their mixtures.
It is further preferred from the viewpoint of solution stability that the total concentration of organic solvents is 75% by weight or more, more preferably 80% by weight or more, further more preferably 85% by weight or more, calculated to the total weight of composition A.
It is further preferred from the viewpoint of dyeing intensity that the total concentration of organic solvents is 98% by weight or less, more preferably 95% by weight or less, further more preferably 92% by weight or less, calculated to the total weight of composition A.
For attaining the above-mentioned effects, it is preferred that the total concentration of organic solvents is in the range of 75% to 98% by weight, more preferably 80% to 95% by weight, further more preferably in the range of 85% to 92% by weight, calculated to the total weight of composition A.

**Hair dyeing oil**

Optionally, composition A is a hair dyeing oil, preferably a paste-like composition, more preferably having a

viscosity in the range of 10,000 mPas to 500,000 mPas, more preferably in the range of 20,000 mPas to 400,000 mPas, still more preferably in the range of 30,000 mPas to 250,000 mPas, measured by cone-plate viscometry at 25°C and atmospheric pressure.

For this purpose, composition A may comprise lipophilic compound(s) according to group e) up to a total concentration of 90% by weight.

Preferably, the paste-like composition A is anhydrous, from the viewpoint of dye stability.

**Surfactants as compounds according to g)**

Compositions A and/or B may further comprise one or more surfactant(s) as compound according to group g), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s), from the viewpoint of stabilizing the composition and improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of $C_{10}$ to $C_{22}$ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of $C_{12}$ to $C_{22}$ or surfactants having a tertiary amine group and at least one alkyl chain having a carbon chain length in the range of $C_{12}$ to $C_{22}$ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of each of the compositions A and/or B, from the viewpoint of enhancing wettability of keratin fibers, physical stability, and mixability with other compositions.

**Thickening polymers**

From the viewpoint of cosmetic safety, it is further preferred that the compositions A A and/or B comprise one or more thickening polymer.

Composition A and/or B may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH ranges between 7 and 12 having a viscosity of at least 1,000 mPa•s measured at a polymer concentration of 1% by weight in water at 25°C determined by cone plate viscometry at 25°C under atmospheric conditions, calculated to the total weight of the composition, determined by a Brookfield viscometer, such as at 10 rpm for 1 min, with an appropriate spindle at 25°C. pH measurements are suitably conducted with a glass electrode at 25°C under atmospheric conditions.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethylmethylcellulose, and alkylated hydroxyl celluloses such as ($C_2$-$C_8$)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers in compositions A and/or B is 0.1% by weight or more, more preferably 0.25% by weight or more, more preferably 0.5% by weight or more, calculated to the total weight of each of the compositions A, and/or B, from the viewpoint of providing sufficient viscosity to the composition.

Preferably, the total concentration of thickening polymers in compositions A and/or B is 15% by weight or less, more preferably 12% by weight or more, further more preferably 10% by weight or less, calculated to the total weight of each of the compositions A and/or B, from the viewpoint of providing sufficient viscosity to the composition and cost of goods.

For attaining the above-mentioned effects, it is preferred that the total concentration of thickening polymers in compositions A and/or B is in the range of 0.1% to 15% by weight, preferably in the range of 0.25% to 12% by weight, more preferably in the range of 0.5% to 10% by weight, calculated to the total weight of each of the compositions A and/or B.

It is preferred from the viewpoint of cosmetic safety that the composition of the present invention, in case that it is aqueous, has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4 and measurements are conducted at 10 rpm for 1 min.

## Oxidizing agents

Compositions A and/or B may comprise one or more oxidizing agent(s), preferably hydrogen peroxide.

Preferably, composition A comprises one or more oxidizing agent being solid at 25°C and atmospheric pressure, more preferably one or more peroxide being solid at 25°C and atmospheric pressure, still more preferably it comprises a metal peroxide such as sodium peroxide, potassium peroxide, magnesium peroxide, melamine peroxide, urea peroxide, and/or their mixtures.

The preferred oxidizing agent for composition B is hydrogen peroxide, from the viewpoint of dyeing performance.

It is further preferred from the viewpoint of dyeing performance that the concentration of oxidizing agents in the compositions A and/or B is 0.1% by weight or more, more preferably 0.25% by weight or more, further more preferably 1% by weight or more, calculated to the total weight of each of the compositions A and/or B.

It is further preferred from the viewpoint of product performance and user safety that the concentration oxidizing agents in compositions A and/or B is 20% by weight or less, more preferably 15% by weight or less, further more preferably 12% by weight or less, calculated to the total weight of each of the compositions A and/or B.

For attaining the above-mentioned effects, it is preferred that the concentration of oxidizing agents in compositions A and/or B is in the range of 0.1% to 20% by weight, more preferably in the range of 0.25% to 15% by weight, further more preferably in the range of 1% to 12% by weight, calculated to the total weight of each of the compositions A and/or B.

## Dyeing product

The present invention is also directed to a dyeing product for keratin fibers, preferably human keratin fibers, more preferably human hair, comprising:

- composition A as defined above,
- a composition B, preferably comprising one or more oxidizing agent(s).

Preferably, composition B is an aqueous composition. The term 'aqueous' denotes a composition comprising 50% by weight of water or more, calculated to the total weight of the composition.

Preferably, from the viewpoint of stability, the composition B has a pH in the range of 1 to 7, preferably in the range of 1.5 to 5, further more preferably in the range of 2 to 4.5

It is further preferred from the viewpoint of cosmetic safety that one or more oxidizing agent(s) of composition B is hydrogen peroxide.

It is further preferred from the viewpoint of dyeing performance that the concentration of oxidizing agents, preferably the concentration of hydrogen peroxide, in the composition B is 0.1% by weight or more, more preferably 0.25% by weight or more, further more preferably 1% by weight or more, calculated to the total weight of composition B.

It is further preferred from the viewpoint of product performance and user safety that the concentration oxidizing agents, preferably the concentration of hydrogen peroxide, in the composition B is 20% by weight or less, more preferably 15% by weight or less, further more preferably 12% by weight or less, calculated to the total weight of composition B.

For attaining the above-mentioned effects, it is preferred that the concentration of oxidizing agents, preferably the concentration of hydrogen peroxide, in the composition B is in the range of 0.1% to 20% by weight, more preferably

in the range of 0.25% to 15% by weight, further more preferably in the range of 1% to 12% by weight, calculated to the total weight of composition B.

Composition B preferably is an emulsion, thickened gel, or a combination thereof, from the viewpoint of cosmetic safety as well as user friendliness. It may comprise lipophilic compound(s) according to group e) and/or surfactant(s) according to group g) and/or one or more thickening polymer(s) as defined above.

It is preferred from the viewpoint of forming a stable composition and user friendliness that the total concentration of compounds according to group e) is 1% by weight or more, more preferably 2% by weight or more, further more preferably 3% by weight or more, calculated to the total weight of composition B.

It is preferred from the viewpoint of forming a stable composition that the total concentration of compounds according to group e) is 20% by weight or less, more preferably 15% by weight or less, further more preferably 12% by weight or less, calculated to the total weight of composition B.

For attaining the above-mentioned effects, the total concentration of compounds according to group e) is in the range of 1% to 20% by weight, preferably in the range of 2% to 15% by weight, more preferably in the range of 3% to 12% by weight, calculated to the total weight of composition B.

Composition B may further comprise one or more surfactant(s) as compound according to group g), as defined above for the composition A of the present invention above.

Suitable concentration ranges for surfactants are in the range of 0.1% to 10% by weight, calculated to the total weight of composition B, from the viewpoint of enhancing wettability of keratin fibers, physical stability, and mixability with other compositions.

From the viewpoint of cosmetic safety, it is further preferred that composition B comprises one or more thickening polymer, as defined for the composition of the present invention above.

Preferably, the total concentration of thickening polymers in composition B is 0.1% by weight or more, more preferably 0.25% by weight or more, more preferably 0.5% by weight or more, calculated to the total weight of composition B, from the viewpoint of providing sufficient viscosity to composition B.

Preferably, the total concentration of thickening polymers in composition B is 15% by weight or less, more preferably 12% by weight or more, further more preferably 10% by weight or less, calculated to the total weight of composition B, from the viewpoint of providing sufficient viscosity to composition B and cost of goods.

For attaining the above-mentioned effects, it is preferred that the total concentration of thickening polymers in composition B is in the range of 0.1% to 15% by weight, preferably in the range of 0.25% to 12% by weight, more preferably in the range of 0.5% to 10% by weight, calculated to the total weight of composition B.

It is preferred from the viewpoint of cosmetic safety that composition B has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4.

It is further preferred from the viewpoint of stability of the ready-to-use mixture that the mixing ratio by weight of compositions A and B is 1:1 or more, preferably 1:2 or more, more preferably 1:4 or more, still more preferably 1:5 or more.

It is further preferred from the viewpoint of stability of the ready-to-use mixture and cosmetic appearance that the mixing ratio by weight of compositions A and B is 1:20 or less, preferably 1:15 or less, more preferably 1:12 or less, still more preferably 1:10 or less.

For attaining the above-mentioned effects, it is preferred that the mixing ratio by weight of compositions A and B is in the range of 1:1 to 1:20, preferably in the range of 1:2 to 1:15, more preferably in the range of 1:4 to 1:12, still more preferably in the range of 1:5 to 1:10.

Preferably, composition B comprises one or more alkylamine salt(s) or alkanolamine salt(s), preferably according to the following general structure

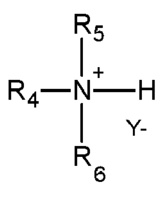

wherein $R_4$, $R_5$, and $R_6$ are independently selected from H, linear $C_1$-$C_6$ alkyl which may be substituted with one hydroxyl group, or branched $C_3$-$C_{12}$ alkyl or alkanol, wherein at least one of $R_4$, $R_5$, or $R_6$ is different from H, and Y- is an anion, from the viewpoint of dyeing intensity.

Suitably, the anion Y- is sulfate, hydrochloride, chloride, hydrobromide, bromide, nitrate, phosphate, hydrogen phosphate, dihydrogen phosphate, methosulfate, citrate, succinate, tartrate, lactate, tosylate, benzoate, benzenesulfonate, acetate, and/or their mixtures

Suitably, one or more alkylamine salt(s) or alkanolamine salt(s) is/are salts of mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or their mixtures, more preferably it/they is/are mono- and/or diethanolamine salt(s), and/or their mixtures.

Suitably, the total concentration of alkylamine salt(s) or alkanolamine salt(s) in composition B is in the range 0.1% to 15% by weight, preferably in the range of 0.2% to 12% by weight, more preferably in the range of 0.5% to 10% by weight, more preferably in the range of 0.5% to 8% by weight, calculated to the total weight of composition B.

**Method for dyeing**

The present invention is also directed to a method for dyeing keratin fibers, preferably human keratin fibers, more preferably human hair, comprising the steps of:

i) mixing the composition A as defined above with an aqueous composition, preferably with composition B as defined in any of the claims 12 to 15, to yield a ready-to-use composition having a pH in the range of 7 to 12,

ii) applying the ready-to-use composition onto keratin fibers and leaving it for a time period in the range of 1 min to 60 min,

iii) optionally rinsing-off the keratin fibers and optionally drying the keratin fibers.

If composition A comprises oxidizing agents, then composition B may be free of oxidizing agents.

The ready-to-use composition is applied to keratin fibers and preferably left for a time period of 1 min to 60 min as defined in step ii). Further preferred time ranges for step ii) are 5 min to 45 min, more preferred ranges are 10 min to 35 min, from the viewpoint of sufficiently dyeing.

Optionally, heat may be applied while leaving the composition A or the ready-to-use composition onto keratin fibers. Suitable temperature ranges are 30°C to 50°C.

Preferably, the mixing ratio by weight of compositions A and B in step i) is in the range of 1:1 to 1:20, preferably in the range of 1:2 to 1:15, more preferably in the range of 1:4 to 1:12, still more preferably in the range of 1:5 to 1:10.

Thus, the disclosure of the present invention also relates to a method for dyeing keratin fibers, preferably human keratin fibers, more preferably human hair, comprising the steps of:

i) mixing the composition A as defined above with an aqueous composition B comprising hydrogen peroxide, to yield a ready-to-use composition having a pH in the range of 7 to 12,

ii) applying the ready-to-use composition onto keratin fibers and leaving it for a time period in the range of 1 min to 60 min,

iii) optionally rinsing-off the keratin fibers and optionally drying the keratin fibers,

wherein the mixing ratio by weight of compositions A and B in step i) is in the range of 1:1 to 1:20, preferably in the range of 1:2 to 1:15, more preferably in the range of 1:4 to 1:12, still more preferably in the range of 1:5 to 1:10.

The present disclosure is also directed to <1> a dyeing composition A for keratin fibers, preferably for human keratin fibers, more preferably for human hair, comprising

a) 1,4-diamino-2-methoxymethyl-benzene, and/or its salt(s), and

b) one or more alkalizing agent(s),

wherein the total concentration of water is 10% by weight or less, calculated to the total weight of composition A.

<2> The composition according to clause <1> characterized in that one or more compound(s) according to group a) is/are sulfate salt, hydrochloride salt, hydrobromide salt, nitrate salt, phosphate salt, hydrogen phosphate salt, dihydrogen phosphate salt, methosulfate salt, citrate salt, succinate salt, tartrate salt, lactate salt, tosylate salt, benzenesulfonate salt, acetate salt, and/or their mixtures, preferably it/they is/are a sulfate salt and/or a hydrochloride salt, and/or their mixtures, more preferably it is a sulfate salt.

<3> The product according to any of the clauses <1> to <2> characterized in that the total concentration of

compound(s) according to group a), preferably the total concentration of 1,4-diamino-2-methoxymethyl-benzene, and/or its salt(s), in the range of 0.01% to 65% by weight, preferably in the range of 0.03% to 50% by weight, more preferably in the range of 0.05% to 30% by weight, still more preferably in the range of 0.1% to 20% by weight, calculated to the total weight of composition A.

<4> The composition according to any of the clauses <1> to <3> characterized in that it comprises one or more inorganic and/or organic alkalizing agent(s), and/or their salt(s), and/or their mixtures.

<5> The composition according to any of the clauses <1> to <4> characterized in that it comprises one or more inorganic alkalizing agent(s), more preferably they are metasilicates, disilicates, carbonates, bicarbonates, and/or phosphates, and/or their metal salts such as alkali or earth alkaline salts, and/or their mixtures, preferably it is sodium metasilicate, trisodium phosphate and/or tripotassium phosphate, and/or their mixtures.

<6> The composition according to any of the clauses <1> to <5> characterized in that it comprises one or more organic alkalizing agent(s) such as one or more organic alkyl amine(s) and/or alkanol amine(s) and/or their salt(s) according to the following general structure:

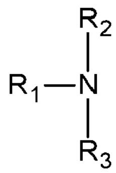

wherein $R_1$, $R_2$, and $R_3$ are independently selected from H, linear $C_1$-$C_6$ alkyl which may be substituted with one hydroxyl group, or branched $C_3$-$C_{12}$ alkyl or alkanol, wherein at least one of $R_1$, $R_2$, or $R_3$ is different from H, and/or their mixtures.

<7> The composition according to any of the clauses <1> to <6> characterized in that one or more organic alkalizing agent(s) is/are selected from alkyl and/or alkanolamine(s), more preferably they/it is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane, and/or their mixtures, preferably is/they is/are selected from monoethanolamine, 2-amino-2-methylpropanol, tris-(hydroxymethyl)-aminomethane, and/or their mixtures.

<8> The composition according to any of the clauses <1> to <7> characterized in that the total concentration of alkalizing agents, preferably of inorganic or organic alkalizing agents, is in the range of 1% to 80% by weight, preferably in the range of 2% to 70% by weight, more preferably in the range of 5% to 65% by weight, more preferably in the range of 10% to 65% by weight, calculated to the total weight of composition A.

<9> The composition according to any of the clauses <1> to <8> characterized in that it comprises one or more oxidative dye precursor or oxidative dye coupler different from group a) as compound(s) according to group c), preferably 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorophenol, 1,3-bis-(2,4-diaminophenoxy)-propane, 2-bis(2-hydroxyethyl)-aminotoluene, 2-amino-5-methylphenol, resorcinol, 2-methylresorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 2,4-diaminophenoxyethanol, 1,3-diamino-benzene, 1-amino-3-(2'-hydroxyethylamino)benzene, 1-amino-3-[bis(2'-hydroxy-ethyl) amino]benzene, $\alpha$-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)-benzene, 2,4,5,6-tetraaminopyrimidine, 2,5,6-triamino-5-pyrimidinol, and/or their salt(s), and/or their mixtures, preferably one or more compound(s) according to group c) is/are selected from 4-chlororesorcinol, 2-methylresorcinol, 1,3-bis-(2,4-diaminophenoxy)-propane, 2-methyl-5-amino-6-chlorophenol, 2-amino-3-hydroxypyridine, 5-amino-6-chloro-o-cresol, 3-amino-2,4-dichlorophenol, and/or their salt(s), and/or their mixtures, more preferably 1,3-bis-(2,4-diamino-phenoxy)-propane, 2,4-diaminophenoxyethanol, 2-amino-4-hydroxyethylaminoanisole, 5-amino-6-chloro-o-cresol, 3-amino-2,4-dichlorophenol, and/or their salt(s), and/or their mixtures.

<10> The composition according to clause <9> characterized in that the total concentration of one or more compound(s) according to group c) is in the range of 0.01% to 20% by weight, preferably in the range of 0.05% to 15% by weight, more preferably in the range of 0.08% to 10% by weight, still more preferably in the range of 0.08% to 5% by weight, still further more preferably in the range of 0.08% to 3.5% by weight, calculated to the total weight of composition A.

<11> The composition according to any of the clauses <8> to <10> characterized in that the weight ratio of compound(s) according to group a) to compound(s) according to group c) is in the range of 0.2 to 5, preferably in the range of 0.3 to 3, more preferably in the range of 0.5 to 2.

<12> The composition according to any of the clauses <1> to <11> characterized in that the total concentration of carbonates, hydrogen carbonates, bicarbonates, percarbonates, borates, and perborates in composition A is less than 10% by weight, preferably less than 5% by weight, more preferably less than 1% by weight, still more preferably less than 0.1% by weight, calculated to the total weight of composition A, still more preferably it is free of carbonates, hydrogen carbonates, bicarbonates, and percarbonates.

<13> The composition according to any of the clauses <1> to <12> characterized in that it comprises one or more direct dye(s), preferably HC Blue 18, HC Red 18, HC Yellow 16, Disperse Black 9, Acid Yellow 1, 2-amino-6-chloro-4-nitrophenol, and/or their salt(s), and/or their mixtures.

<14> The composition according to clause <13> characterized in that the total concentration of direct dyes in composition A is in the range of 0.01% to 20% by weight is in the range of 0.05% to 15% by weight, more preferably in the range of 0.1% to 10% by weight, calculated to the total weight of composition A.

<15> The composition according to any of the clauses <1> to <14> characterized in that it comprises one or more pulverulent excipient as compound(s) according to group d).

<16> The composition according to clause <15> characterized in that it comprises an organic and/or an inorganic pulverulent as compound(s) according to group d).

<17> The composition according to any of the clauses <15> to <16> characterized in that one or more compound(s) according to group d) is/are diatomaceous earth, kaolin, bentonite, starch especially corn, tapioca, rice, wheat and potato, nylon powder, montmorillonit, gypsum, sawdust and perlite, more preferably it is corn starch, and/or their mixtures.

<18> The composition according to any of the clauses <15> to <17> characterized in that the total concentration of compound(s) according group d), more preferably the total concentration of organic and/or an inorganic pulverulent excipients, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

<19> The composition according to any of the clauses <1> to <18> characterized in that it is a solid composition at 25°C and atmospheric pressure, preferably a powder composition or a pellet composition or a tablet composition or capsule composition, more preferably a hair dyeing powder composition or a hair dyeing pellet composition or a hair dyeing tablet composition, still more preferably it is a hair dyeing powder composition.

<20> The composition according to clause <19> characterized in that it is an anhydrous powder composition, preferably an anhydrous hair dyeing powder composition.

<21> The composition according to any of the clauses <1> to <20> characterized in that it comprises one or more lipophilic compound(s) as compound(s) according to group e), preferably selected from $C_{12}$ to $C_{22}$ fatty alcohols, esters of $C_3$ to $C_{22}$ alcohols with $C_{12}$ to $C_{22}$ fatty acids, $C_8$ to $C_{22}$ fatty acids, vegetable oils, and/or silicones, and/or hydrocarbon-based products, and/or their mixtures, from the viewpoint of cosmetic compatibility.

<22> The composition according to clause <21> characterized in that one or more $C_{12}$ to $C_{22}$ fatty alcohol(s) as compound(s) according to e) is/are myristyl alcohol, cetyl alcohol, stearyl alcohol, behenyl alcohol, and cetearyl alcohol, one or more esters of $C_3$ to $C_{22}$ alcohols with $C_{12}$ to $C_{22}$ fatty acids as compound(s) according to e) is/are isopropyl myristate, isopropyl palmitate, and myristyl myristate, one or more $C_8$ to $C_{22}$ fatty acids as compound(s) according to e) is/are oleic acid, linoleic acid, and palmitic acid, one or more vegetable oil(s) as compound(s) according to e) is/are olive oil, almond oil, sunflower oil, and argan oil, one or more silicone(s) as compound(s) according to e) is/are non-aminated and/or aminated silicones, and/or their mixtures.

<23> The composition according to any of the clauses <21> to <22> characterized in that the total concentration of compounds according to group e) is in the range of 1% to 20% by weight, preferably in the range of 2% to 15% by weight, more preferably in the range of 3% to 12% by weight, calculated to the total weight of composition A.

<24> The composition according to any of the clauses <1> to <14> characterized in that is a liquid composition at 25°C and atmospheric pressure and comprises one or more organic solvent(s) as compound(s) according to group f), preferably one more mono-, di-, and trivalent alcohols, and/or their mixtures as one or more compound(s) according to f).

<25> The composition according to clause <24> characterized in that one or more compound(s) according to group f) is/are ethanol, n-propanol, isopropanol, propylene glycol, ethylene glycol, benzyl alcohol, phenoxyethanol, and glycerol, and/or their mixtures.

<26> The composition according to any of the clauses <24> to <25> characterized in that the total concentration of one or more compound(s) according to group f) is in the range of 75% to 98% by weight, more preferably 80% to 95% by weight, further more preferably in the range of 85% to 92% by weight, calculated to the total weight of composition A.

<27> The composition according to any of the clauses <1> to <18> and/or <21> to

<22> characterized in that composition A is a hair dyeing oil.

<28> The composition according to clause <27> characterized in that composition A has a paste-like viscosity, more preferably it has a viscosity in the range of 10,000 mPas to 500,000 mPas, more preferably in the range of 20,000

mPas to 400,000 mPas, still more preferably in the range of 30,000 mPas to 250,000 mPas, measured by cone-plate viscometry at 25°C and atmospheric pressure.

<29> The composition according to any of the clauses <27> to <28> characterized in that the total concentration of one or more compound(s) according to group e) is 90% by weight or less, preferably in the range of 50% to 90% by weight, calculated to the total weight of composition A.

<30> The composition according to any of the clauses <1> to <29> characterized in that it comprises one or more surfactant(s) as compound according to group g), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s).

<31> The composition according to clause <30> characterized in that one or more compound(s) according to group g) is/are one or more anionic surfactant(s), preferably selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts, one or more alkyl sulfate(s) preferably ethoxylated alkyl ether sulfate surfactants or mixtures thereof, and/or salts thereof, having an alkyl chain length of $C_{10}$ to $C_{22}$ and an ethoxylation degree from 1 to 50, one or more non-ionic surfactant(s) selected from alkyl glycosides, alkyl polyglycosides, ethoxylated triglycerides, ethoxylated fatty alcohols, ethoxylated fatty acid esters, and/or their mixtures, one or more cationic surfactant(s) selected from quaternary ammonium surfactants having a carbon chain length in the range of $C_{12}$ to $C_{22}$ or surfactants having a tertiary amine group and at least one alkyl chain having a carbon chain length in the range of $C_{12}$ to $C_{22}$ such as alkylamidoalkylamine surfactants, and/or their salts, and/or their mixtures, and one or more amphoteric/zwitterionic surfactants of betaine type, and/or their salt(s), and/or their mixtures.

<32> The composition according to any of the clauses <30> to <31> characterized in that the total concentration of surfactants is in the range of 0.1% to 10% by weight, calculated to the total weight composition A.

<33> The composition according to any of the clauses <1> to <32> characterized in that it comprises one or more thickening polymer(s), preferably one or more thickening polymer(s) selected from non-ionic thickening polymers and/or anionic thickening polymers, and/or their mixtures.

<34> The composition according to clause <33> characterized in that one or more thickening polymer(s) is/are a non-ionic thickening polymer, preferably a cellulose-based polymers, more preferably methylcellulose, hydroxymethylcellulose, hydroxyethylcellulose, hydroxypropylcellulose, hydroxyethyl-methylcellulose, and alkylated hydroxyl celluloses such as ($C_2$-$C_8$)-alkylcelluloses or cetyl hydroxyethylcellulose, an anionic thickening polymer, preferably a naturally-based anionic polymers and/or synthetic anionic polymers, more preferably xanthan gum, dehydroxanthan gum, hydroxypropylxanthan gum, carboxymethyl cellulose and starch based polymers such as vegetable starch and/or their synthetically modified derivatives such as hydroxypropyl starch phosphate, alginic acids, sodium alginates, ammonium alginates, calcium alginates, gum arabic, and guar gum, and/or an associative thickening polymer such as acrylates/steareth-30 methacrylate copolymer.

<35> The composition according to any of the clauses <33> to <34> characterized in that one or more thickening polymer is a natural anionic polymer, more preferably xanthan gum and/or dehydroxanthan gum.

<36> The composition according to any of the clauses <33> to <35> characterized in that the total concentration of thickening polymers is in the range of 0.1% to 15% by weight, preferably in the range of 0.25% to 12% by weight, more preferably in the range of 0.5% to 10% by weight, calculated to the total weight of composition A.

<37> The composition according to any of the clauses <33> to <36> characterized in that it has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4.

<38> The composition according to any of the clauses <1> to <37> characterized in that it comprises one or more oxidizing agent(s) being solid at 25°C and atmospheric pressure, more preferably one or more peroxide being solid at 25°C and atmospheric pressure, still more preferably it comprises a metal peroxide such as sodium peroxide, potassium peroxide, magnesium peroxide, melamine peroxide, urea peroxide, and/or their mixtures.

<39> The composition according to clause <38> characterized in that the total concentration of one or more oxidizing agent being solid at 25°C and atmospheric pressure is in the range of 0.1% to 50% by weight, more preferably in the range of 0.25% to 40% by weight, further more preferably in the range of 1% to 30% by weight, calculated to the total weight composition A.

The present disclosure is also directed to <40> a dyeing product for keratin fibers, preferably human keratin fibers, more preferably human hair, comprising:

- composition A as defined in any of the clauses <1> to <39>,

- a composition B, preferably comprising one or more oxidizing agent(s).

<41> The product according to clause <40> characterized in that composition B has a pH in the range of 1 to 6, preferably in the range of 1.5 to 5, more preferably in the range of 2 to 4.5, and comprises hydrogen peroxide.

<42> The product according to any of the clauses <40> to <41> characterized in that the total concentration of hydrogen peroxide in composition B is in the range of 0.1% to 20% by weight, more preferably in the range of 0.25% to 15% by weight, further more preferably in the range of 1% to 12% by weight, calculated to the total weight of the composition B.

<43> The product according to any of the clauses <40> to <42> characterized in that composition B is an emulsion, thickened gel, or a combination thereof, and comprises one or more lipophilic compound(s) according to group e) and/or one or more surfactant(s) according to group g) and/or one or more thickening polymer(s).

<44> The product according to any of the clauses <40> to <43> characterized in that the total concentration of compounds according to group e) in composition B is in the range of 1% to 20% by weight, preferably in the range of 2% to 15% by weight, more preferably in the range of 3% to 12% by weight, calculated to the total weight of composition B.

<45> The product according to any of the clauses <40> to <44> characterized in that the total concentration of one or more surfactant(s) as compound according to group g) in composition B in the range of 0.1% to 10% by weight, calculated to the total weight of composition B.

<46> The product according to any of the clauses <40> to <45> characterized in that the total concentration of thickening polymers in composition B is in the range of 0.1% to 15% by weight, preferably in the range of 0.25% to 12% by weight, more preferably in the range of 0.5% to 10% by weight, calculated to the total weight of composition B.

<47> The product according to any of the clauses <40> to <46> characterized in that composition B has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions, preferably measured with a Brookfield viscometer with spindle #4.

<48> The product according to any of the clauses <40> to <47> characterized in that the mixing ratio by weight of compositions A and B is in the range of 1:1 to 1:20, preferably in the range of 1:2 to 1:15, more preferably in the range of 1:4 to 1:12, still more preferably in the range of 1:5 to 1:10.

<49> The product according to any of the clauses <40> to <48> characterized in that composition B comprises one or more alkylamine salt(s) or alkanolamine salt(s), preferably according to the following general structure

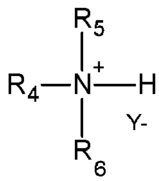

wherein $R_4$, $R_5$, and $R_6$ are independently selected from H, linear $C_1$-$C_6$ alkyl which may be substituted with one hydroxyl group, or branched $C_3$-$C_{12}$ alkyl or alkanol, wherein at least one of $R_4$, $R_5$, or $R_6$ is different from H, and Y- is an anion.

<50> The product according to clause <49> characterized in that the anion Y- is sulfate, hydrochloride, chloride, hydrobromide, bromide, nitrate, phosphate, hydrogen phosphate, dihydrogen phosphate, methosulfate, citrate, succinate, tartrate, lactate, tosylate, benzoate, benzenesulfonate, acetate, and/or their mixtures

<51> The product according to any of the clauses <49> to <50> characterized in that one or more alkylamine salt(s) or alkanolamine salt(s) is/are salts of mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or their mixtures, more preferably it/they is/are mono- and/or diethanolamine salt(s), and/or their mixtures.

<52> The product according to any of the clauses <49> to <51> characterized in that the total concentration of alkylamine salt(s) or alkanolamine salt(s) in composition B is in the range 0.1% to 15% by weight, preferably in the range of 0.2% to 12% by weight, more preferably in the range of 0.5% to 10% by weight, more preferably in the range of 0.5% to 8% by weight, calculated to the total weight of composition B.

The present disclosure is also directed to <53> a method for dyeing keratin fibers, preferably human keratin fibers, more preferably human hair, comprising the steps of:

i) mixing the composition A as defined in any of the clauses <1> to <39> with an aqueous composition, preferably with composition B as defined in any of the clauses <40> to <52>, to yield a ready-to-use composition having a pH in the range of 7 to 12,

ii) applying the ready-to-use composition onto keratin fibers and leaving it for a time period in the range of 1 min to 60 min,

iii) optionally rinsing-off the keratin fibers and optionally drying the keratin fibers.

<54> The method according to clause <53> characterized in that the ready-to-use composition is applied to keratin fibers and preferably left for a time period of 1 min to 60 min, more preferably for a time period of 5 min to 45 min, further more preferably for a time period of 10 min to 35 min, as defined in step ii).

<55> The method according to any of the clauses <53> to <54> characterized in that heat is applied while leaving the composition A or the ready-to-use composition onto keratin fibers, preferably in a temperature range of 30°C to 50°C.

<56> The method according to any of the clauses <53> to <55> characterized in that mixing ratio by weight of compositions A and B in step i) is in the range of 1:1 to 1:20, preferably in the range of 1:2 to 1:15, more preferably in the range of 1:4 to 1:12, still more preferably in the range of 1:5 to 1:10.

The following examples are to illustrate the invention, but not to limit it.

**EXAMPLES**

**Table 1**

| **Ingredients composition A** | **Inv. Ex. 1** | **Comp. Ex. 1** | **Inv. Ex. 2** | **Comp. Ex. 2** |
|---|---|---|---|---|
| | **[% by weight]** | | | |
| 1,4-diamino-2-methoxymethyl-benzene sulfate | 2.28 | - | 0.25 | - |
| p-phenylene diamine | - | 0.99 | - | 0.11 |
| 4-Chlororesorcinol | 1.32 | 1.32 | - | - |
| 5-Am ino-6-chloro-o-cresol | - | - | 0.16 | 0.16 |
| Trisodium phosphate | 55.0 | 55.0 | 55.0 | 55.0 |
| Sodium metasilicate | 3.0 | 3.0 | 3.0 | 3.0 |
| Corn starch | Ad 100.0 | | | |
| | | | | |
| Mixing ratio by weight comp. A:B | 1:10 | 1:10 | 1:10 | 1:10 |
| | | | | |
| **Wash fastness** | | | | |
| Directly upon dyeing - 20 x washing (ΔΔE) | 8.4 | 10.8 | 19.6 | 22.9 |

The differences in weight concentration of 1,4-diamino-2-methoxymethyl-benzene sulfate and p-phenylene diamine resulted due to their differences in molar mass while using equimolar concentrations (0.01 mol).

As a result, 1,4-diamino-2-methoxymethyl-benzene had a better fastness in comparison to p-phenylene diamine.

**Composition B**

| | **% by weight** |
|---|---|
| Hydrogen peroxide | 4.5 |
| Phosphoric acid | ad pH 2.0 |
| Water | ad 100.0 |

**Table 2**

| **Ingredients composition A** | **Inv. Ex. 3** | **Inv. Ex. 4** | **Inv. Ex. 5** |
|---|---|---|---|
| | **[% by weight]** | | |
| 1,4-diamino-2-methoxymethyl-benzene | 8.66 | 8.66 | 8.66 |

(continued)

| Ingredients composition A | Inv. Ex. 3 | Inv. Ex. 4 | Inv. Ex. 5 |
|---|---|---|---|
| | [% by weight] | | |
| 4-Chlorresorcinol | 5.0 | 5.0 | 5.0 |
| Trisodium phosphate | 50.0 | - | - |
| Sodium disilicate | - | 50.0 | - |
| Sodium metasilicate | - | - | 22.2 |
| Kaolin | Ad 100.0 | | |
| | | | |
| Mixing ratio by weight comp. A:B | 1:10 | 1:10 | 1:10 |
| | | | |
| **Color results** | | | |
| Upon preparation (ΔE) | 54 | 55 | 52 |
| Mixture 30 min upon preparation (ΔE) | 50 | 52 | 50 |
| Upon preparation - 30 min upon preparation (ΔΔE) | 4 | 3 | 2 |

**Composition B**

| | % by weight |
|---|---|
| Hydrogen peroxide | 4.5 |
| Monoethanolamine sulfate | 2.0 |
| Phosphoric acid | ad pH 2.0 |
| Water | ad 100.0 |

The compositions above had excellent dyeing performance directly upon preparation of the ready-to-use mixture and after 30 min storage of the ready-to-use mixture.

## Methods

### Compositions and their storage

The compositions of table 1 were prepared by conventional mixing methods in a ball mill with alumina ceramic grinding balls (Turbula, System Schatz, W.A. Bachofen AG, Maschinenfabrik Schweiz). Directly upon preparation, dyeing experiments were carried out as detailed below.

### Hair dyeing

The compositions A of table 1 were mixed with composition B in the weight ratio of 1:10 to prepare ready-to-use compositions having a pH in the range of 9.5 to 10.0.

Goat hairstreaks (21 cm, 2 g per bundle) were treated with 2 g of the ready-to-use dyeing compositions from above either directly upon preparation or after 30 min storage at 25°C for 20 min at 40°C. The streaks were then rinsed off and dried. The colormetric data were obtained with a color-difference meter (Datacolor 600) in the CIE colorimetric system (L*,a*,b*). Color differences (ΔE) were calculated by the following formula.

$$\Delta E = \sqrt{(L_1^* - L_0^*)^2 + (a_1^* - a_0^*)^2 + (b_1^* - b_0^*)^2}$$

### Wash fastness

Goat hair streaks (21 cm, 2 g per bundle) were permed with 2 g per streak of acommercially available perm

under the trade Name Goldwell Structure and Shine. After that the perm was fixed with 3% by weight hydrogen peroxide solution was 20 min at room temperature. The streaks were dyed according to the protocol above. To the dyed hair streaks commercial shampoo was applied available under the trade name Goldwell Deep Cleansing Shampoo and it was foamed up with lukewarm water for 30 s. The hair streaks were then rinsed off. These steps were repeated for 20 times. After treatment, the hairstreaks were blow-dried and the remaining color was measured. ΔΔE was calculated as the color difference between freshly colored hair and 20 times washed streaks.

The following examples are within the scope of the present invention.

**Inventive Example 6**

**Composition A**

| | **% by weight** |
|---|---|
| 1,4-diamino-2-methoxymethyl-benzene sulfate | 0.097 |
| 5-Am ino-6-chloro-o-cresol | 0.1 |
| 2-methylresorcinol | 0.1 |
| HC Blue 18 | 0.05 |
| HC Red 18 | 0.08 |
| HC Yellow 16 | 0.02 |
| Trisodium phosphate | 55.0 |
| Corn starch | ad 100.0 |

Composition B is as disclosed under table 2. Mixing weight ratio of compositions A:B is 1:10.

## Claims

1. A dyeing composition A for keratin fibers, preferably for human keratin fibers, more preferably for human hair, comprising

   a) 1,4-diamino-2-methoxymethyl-benzene, and/or its salt(s), and
   b) one or more alkalizing agent(s),

   wherein the total concentration of water is 10% by weight or less, calculated to the total weight of composition A.

2. The composition according to claim 1 **characterized in that** the total concentration of water in composition A is 5% by weight or less, preferably 1% by weight or less, more preferably 0.1% by weight or less, calculated to the total weight of composition A, still more preferably composition A is an anhydrous composition.

3. The composition according to any of the preceding claims **characterized in that** the total concentration of one or more compound(s) according to group a) is/are in the range of 0.01% to 65% by weight, preferably in the range of 0.03% to 50% by weight, more preferably in the range of 0.05% to 30% by weight, still more preferably in the range of 0.1% to 20% by weight, calculated to the total weight of composition A.

4. The composition according to any of the preceding claims **characterized in that** one or more compound(s) according to group b) is/are one or more inorganic alkalizing agent(s), preferably one or more metal salt of silicate, metasilicate, disilicate, hydroxide, or phosphate, more preferably one or more sodium salt(s) or potassium salt(s) of silicate, metasilicate, disilicate, hydroxide, or phosphate, and/or their mixtures, still more preferably trisodium phosphate or tripotassium phosphate, and/or their mixtures.

5. The composition according to any of the preceding claims **characterized in that** the total concentration of compound(s) according to group b) in composition A is in the range of 1% to 80% by weight, preferably in the range of 2% to 70% by weight, more preferably in the range of 5% to 65% by weight, more preferably in the range of 10% to 65% by weight, calculated to the total weight of composition A.

**6.** The composition according to any of the preceding claims **characterized in that** the total concentration of carbonates, hydrogen carbonates, bicarbonates, percarbonates, borates, and perborates is less than 10% by weight, preferably less than 5% by weight, more preferably less than 1% by weight, still more preferably less than 0.1% by weight, calculated to the total weight of composition A, still more preferably it is free of carbonates, hydrogen carbonates, bicarbonates, and percarbonates.

**7.** The composition according to any of the preceding claims **characterized in that** composition A comprises one or more pulverulent excipient(s), preferably one or more organic or inorganic pulverulent excipient(s), more preferably one or more pulverulent excipient(s) selected from diatomaceous earth, kaolin, bentonite, silicates, starches such as corn starch, tapioca starch, rice starch, wheat starch and potato starch, nylon powder, montmorillonit, gypsum, sawdust and perlite, and/or their mixtures.

**8.** The composition according to any of the preceding claims **characterized in that** composition A is a solid composition at 25°C and atmospheric pressure, preferably a powder composition or a pellet composition or a tablet composition or capsule composition, more preferably a powder hair dyeing composition or a pellet hair dyeing composition or a tablet hair dyeing composition, still more preferably it is a powder hair dyeing composition.

**9.** The composition according to any of the preceding claims **characterized in that** composition A comprises one or more lipophilic compound(s), preferably one or more lipophilic compound(s) being liquid at 25°C and atmospheric pressure, more preferably $C_{12}$ to $C_{22}$ fatty alcohols, esters of $C_3$ to $C_{22}$ alcohols with $C_{12}$ to $C_{22}$ fatty acids, $C_8$ to $C_{22}$ fatty acids, vegetable oils, and/or silicones, and/or hydrocarbon-based products, and/or their mixtures.

**10.** The composition according to any of the claims 1 to 8 and/or 10 **characterized in that** composition A is a hair dyeing oil, preferably having a paste-like composition, more preferably having a viscosity in the range of 10,000 mPas to 500,000 mPas, more preferably in the range of 20,000 mPas to 400,000 mPas, still more preferably in the range of 30,000 mPas to 250,000 mPas, measured by cone-plate viscometry at 25°C and atmospheric pressure.

**11.** A dyeing product for keratin fibers, preferably human keratin fibers, more preferably human hair, comprising:

- composition A as defined in any of the claims 1 to 10,
- a composition B, preferably comprising one or more oxidizing agent(s).

**12.** The product according to claim 11 **characterized in that** composition B is an aqueous composition having a pH in the range of 1 to 6, preferably in the range of 1.5 to 5, more preferably in the range of 2 to 4.5.

**13.** The product according to any of the claims 11 and/or 12 **characterized in that** composition B comprises one or more oxidizing agent(s), preferably hydrogen peroxide, at a concentration in the range of 1% to 20% by weight, more preferably in the range of 2% to 15% by weight, still more preferably in the range of 3% to 12% by weight, calculated to the total weight of composition B.

**14.** The product according to any of the claims 11 to 13 **characterized in that** the mixing ratio by weight of compositions A and B is in the range of 1:1 to 1:20, preferably in the range of 1:2 to 1:15, more preferably in the range of 1:4 to 1:12, still more preferably in the range of 1:5 to 1:10.

**15.** The product according to any of the claims 11 to 14 **characterized in that** composition B comprises one or more alkylamine salt(s) or alkanolamine salt(s), preferably according to the following general structure

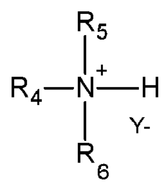

wherein $R_4$, $R_5$, and $R_6$ are independently selected from H, linear $C_1$-$C_6$ alkyl which may be substituted with one hydroxyl group, or branched $C_3$-$C_{12}$ alkyl or alkanol, wherein at least one of $R_4$, $R_5$, or $R_6$ is different from H, and Y- is an anion.

**16.** A method for dyeing keratin fibers, preferably human keratin fibers, more preferably human hair, comprising the steps of:

i) mixing the composition A as defined in any of the claims 1 to 10 with an aqueous composition, preferably with composition B as defined in any of the claims 11 to 14, to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers.

Europäisches Patentamt
European Patent Office
Office européen des brevets

# EUROPEAN SEARCH REPORT

Application Number
EP 21 20 0135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 103 542 A2 (WELLA AG [DE]) 30 May 2001 (2001-05-30) * examples 1, step 3 * ----- | 1-3,5,15 | INV. A61K8/02 A61K8/24 A61K8/41 A61Q5/10 |
| X | WO 2018/039314 A1 (NOXELL CORP [US]) 1 March 2018 (2018-03-01) * example 10 * ----- | 1 | |
| X | WO 2019/120535 A1 (LABORATOIRE BIOSTHETIQUE KOSMETIK GMBH & CO KG [DE]) 27 June 2019 (2019-06-27) * pages 13-14 * * pages 5-7,9,10 * ----- | 1-15 | |
| A | SCHUTTELAAR MARIE L. ET AL: "Cross-elicitation responses to 2-methoxymethyl- p -phenylenediamine in p -phenylenediamine-allergic individuals: Results from open use testing and diagnostic patch testing", CONTACT DERMATITIS: ENVIRONMENTAL AND OCCUPATIONAL DERMATITIS, vol. 79, no. 5, 1 November 2018 (2018-11-01), pages 288-294, XP055893715, US ISSN: 0105-1873, DOI: 10.1111/cod.13078 Retrieved from the Internet: URL:https://api.wiley.com/onlinelibrary/tdm/v1/articles/10.1111%2Fcod.13078> * the whole document * ----- | 1 | TECHNICAL FIELDS SEARCHED (IPC): A61K A61Q |

1 The present search report has been drawn up for all claims

| Place of search | Date of completion of the search | Examiner |
|---|---|---|
| Munich | 11 March 2022 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 20 0135

This annex lists the patent family members relating to the patent documents cited in the above-mentioned European search report. The members are as contained in the European Patent Office EDP file on
The European Patent Office is in no way liable for these particulars which are merely given for the purpose of information.

11-03-2022

| Patent document cited in search report | Publication date | Patent family member(s) | Publication date |
|---|---|---|---|
| EP 1103542 A2 | 30-05-2001 | AT 265420 T | 15-05-2004 |
| | | BR 0005625 A | 17-07-2001 |
| | | DE 19957282 C1 | 17-05-2001 |
| | | EP 1103542 A2 | 30-05-2001 |
| | | ES 2220301 T3 | 16-12-2004 |
| | | JP 4293407 B2 | 08-07-2009 |
| | | JP 4947737 B2 | 06-06-2012 |
| | | JP 2001151739 A | 05-06-2001 |
| | | JP 2009149674 A | 09-07-2009 |
| | | US 6648923 B1 | 18-11-2003 |
| | | US 2003041392 A1 | 06-03-2003 |
| WO 2018039314 A1 | 01-03-2018 | EP 3287120 A1 | 28-02-2018 |
| | | US 2019201308 A1 | 04-07-2019 |
| | | WO 2018039314 A1 | 01-03-2018 |
| WO 2019120535 A1 | 27-06-2019 | EP 3727599 A1 | 28-10-2020 |
| | | US 2020368129 A1 | 26-11-2020 |
| | | WO 2019120535 A1 | 27-06-2019 |

EPO FORM P0459

For more details about this annex : see Official Journal of the European Patent Office, No. 12/82